# EUROPEAN PATENT APPLICATION

(11) **EP 3 782 554 A1**
(43) Date of publication of application: **24.02.2021**
(21) Application number: 20020133.3
(22) Date of filing: 24.03.2020
(51) Int. Cl.: A61B 10/00

(54) **SANITARY CONTAINER**

(30) Priority: 22.03.2019 ES 201930469 U
(71) Applicant: Lambda Diagnostico, S.L.U., 46015 Valencia, Valencia (ES)
(72) Inventor: DIEZ SÁNCHEZ, Juan Carlos, 46015 Valencia (ES)
(74) Representative: De Castro Hermida, José Luis

(57) **Abstract**

Sanitary container comprised of one container (1) with a mouth (2) and a stopper (3), comprising a seal (4) in an intermediate point of the container, that defines a lower part designed to contain a product watertight, and also comprising at least one extension (5) protruding from the mouth (2), which is configured to render the seal (4) useless when the stopper (3) is placed.

It comprises several ways of making the seal (4) and variations in which the product is contained inside removable cartridges (13).

## Description

### FIELD OF THE ART

The present invention refers to a sanitary container for preserving samples, such as biopsies, that require the sample to come into contact with a preservation product.

It has applications in the field of medicine and veterinary.

### STATE OF THE ART

In the practice of medicine, it is frequently necessary to take a tissue sample for analysis, generally a biopsy. This sample needs to be preserved in a container with a product, currently formaldehyde, in a very precise concentration in order to facilitate the analysis.

The traditional procedure implies arranging the product in the container and having it at hand for the surgeon, doctor or veterinarian to place the sample and close the container.

However, formaldehyde has a series of drawbacks, such as the toxicity thereof. Therefore, it is not desirable to be exposed to the air during a long time. In order to do so, containers with two compartments have been designed, an upper and a lower compartment, so that the formaldehyde is in the upper compartment and the medical assistant must press a button or break a seal in order to pour the formaldehyde on the sample.

This solution has two drawbacks: the first one is that the medical assistant may forget to take the required action, so the sample is no longer useful. The second one is that the second compartment modifies the upper surface of the container and affects the weight distribution thereof, so that it becomes more difficult to stack containers, and the number of accidental spillages increase.

The applicant does not know of any other device with the same advantages as the invention.

### BRIEF EXPLANATION OF THE INVENTION

The invention consists of a sanitary container according to the claims.

The sanitary container is the type of container comprising a mouth and a stopper, generally female. In addition, it comprises a seal in an intermediate point of the container, which does not have to correspond to the exact centre. The seal defines a lower part designed to contain a product watertight, and an upper part accessible from the mouth. It also comprises at least one extension resting on the seal and protruding from the mouth. The extension, or extensions, are configured to render the seal useless when the stopper is placed by means of threading, pressure or a different method.

This solution makes it possible to, on the one hand, prevent the product from escaping or being contaminated, and also to make the product come out of the container by merely placing the stopper. This "passive" system is more efficient than previous systems and does not produce any relevant movements of the centre of mass nor of the surface of the stopper. Therefore, it can be handled just as a normal container.

In one embodiment, the extension comprises a thin transversal protrusion that allows the sample to be released from the clamps of the medical assistant when the sample is stuck to them.

In a preferred embodiment, the stopper comprises an extension or seal that allows it to be coupled to the container without affecting the extensions.

Some exemplary embodiments of the seal and the extensions would be:
A seal comprising a series of through-holes blocked by a flexible sheet adhered weakly from the upper part thereof. Thus, as the seal descends and increases the pressure on the lower part thereof, the sheet is peeled off and the two parts of the container become connected.
A seal comprising at least one weak spot, with the bottom of the container comprising one projection. The weak spot defines two parts of the seal, one coinciding with the projection and one coinciding with the extensions. Therefore, when the extensions descend, they create stresses in the seal, which is broken at the weak spot.
A seal that is immobile on the container and configured so that moving the extensions opens up a space for the seal to go through. That is, the extensions carry out the function of a lid in the space already created in the seal, and the lid is removed by the downward movement (towards the bottom of the container) of the extensions.

The seal with the preserving product can be supplied in watertight containers that should be inserted in the container (at the factory or on-site) before inserting the sample and closing the stopper.

Other variations will be described in other parts of the description.

### DESCRIPTION OF THE DRAWINGS

In order to ensure a better understanding of the invention, the following drawings are included.
Figure 1.- shows a first exemplary embodiment of the container of the invention in the initial position (left) and the final position (right).
Figure 2.- shows a second exemplary embodiment of the container of the invention in the initial position (left) and the final position (right).
Figure 3.- shows a third exemplary embodiment of the container of the invention in the initial position (left) and the final position (right).
Figure 4.- shows a fourth exemplary embodiment of the container of the invention in the initial position (left) and the final position (right), in which the product is inside a cartridge.

### EMBODIMENTS OF THE INVENTION

Hereinafter, a brief description of an embodiment of the invention is provided, for purely illustrative purposes and without limitation.

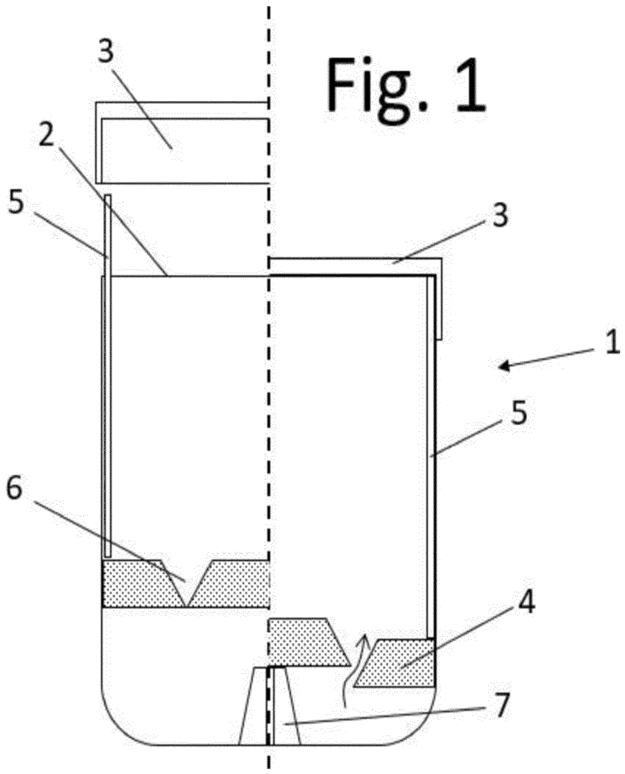

Figure 1 shows an exemplary embodiment corresponding to a container formed by a container (1) with an upper mouth (2) closed by a stopper (3) moving downward, which is generally threaded. The stopper (3) will preferably be female in order to facilitate the rest of the operations.

The container (1) comprises a mobile seal (4) that divides it in two parts, an upper and lower part isolated between them. The preserving product is placed in the lower part, which is generally formaldehyde in a suitable concentration. It is not obligatory for the lower part to be completely full of the product. In fact, it is advisable that it is not completely full since liquids tend to be incompressible.

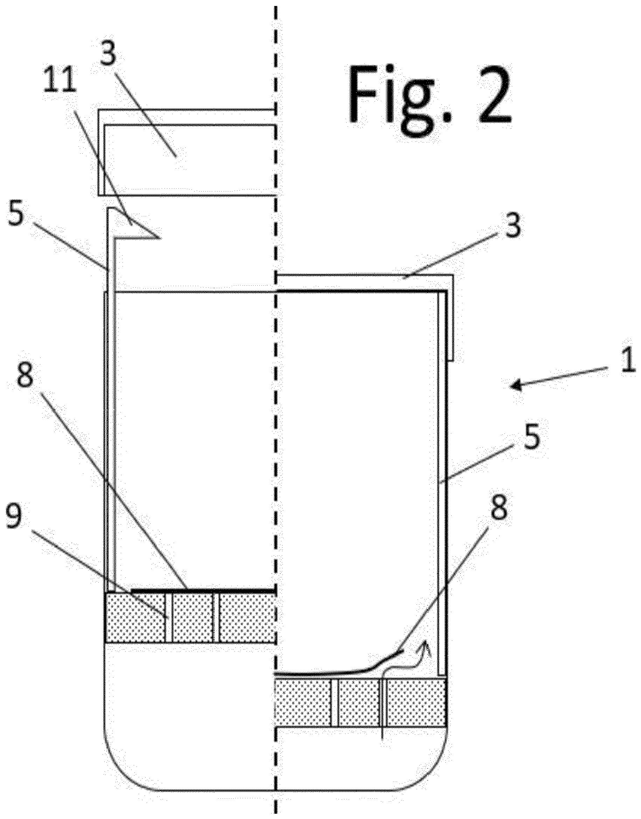

The upper part can contain distilled water, a saline solution or nothing at all. The seal (4) comprises vertical extensions (5) towards the mouth (2), which protrude from it while the container is not adequately closed, with the sample. Therefore, in the open position of Figure 1, the extensions (5) protrude from the mouth, while in the closed position of Figure 2, the stopper (3) has made the extensions (5) and the seal (4) descend in order to achieve a watertight closure.

In order for said descent to happen, it is necessary for the movement to break the seal (4), or at least render it useless. In order to do so, there are several solutions, some of which are listed in some examples below.

In Figure 1, the seal (4) comprises a weak spot (6) while the bottom of the container (1) comprises a projection (7). As the seal (4) descends with the stopper (3), the projection (7) meets the seal (4), which is fractured.

In Figure 2, the seal (4) comprises a flexible sheet (8), made of silicone, for example, adhered weakly to the upper surface most proximate to the mouth (2), and the seal (4) comprises through-holes (9). As the seal (4) descends, the increase in pressure on the lower part causes the sheet (8) to separate and both parts of the container (4) to communicate. This embodiment corresponds to a unidirectional valve that requires a bit of pressure in order to operate. The adhesion can be carried out, among other examples, by means of electrostatic, friction, or by attachments on one or more points that are freed by means of pressure. It is advisable to keep the sheet (8) under control to prevent it from returning to the starting position thereof if the upper part of the container (1) comprises any liquids. Otherwise, it will not be possible to guarantee the resulting concentration of the product.

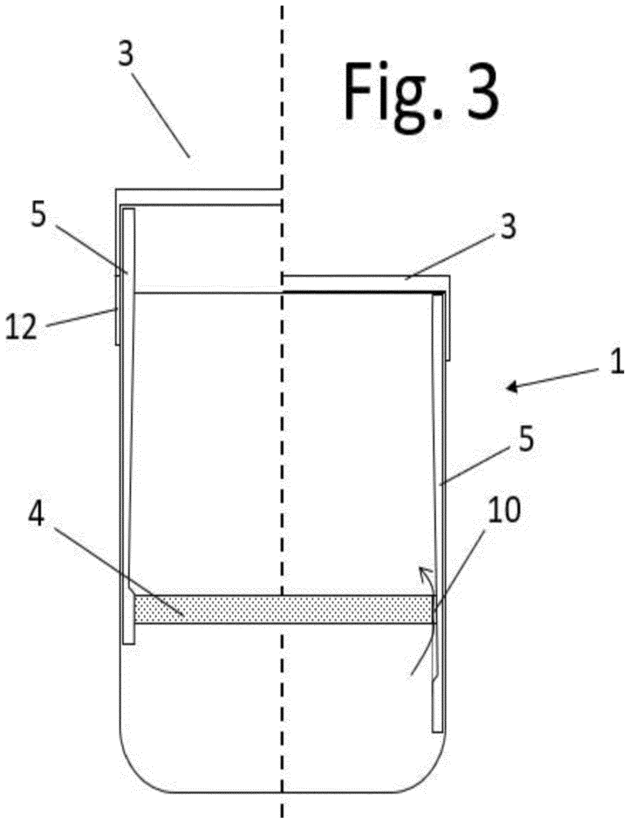

In a third embodiment, the seal (4) does not move along with the extensions (5); instead, the relative movement between them creates a space (10) communicating both parts (Figure 3). This immobilisation can be achieved by means of a projection (7) similar to that of Figure 1, or by making the seal (4) snap fit to the container, so that the strength required to move it is higher than the strength required to move the extensions (5) in relation to the seal (4), thus breaking a small union or adhesion.

The container (1) generally has a constant section since it is the easiest technical solution and also the most common one. However, if it was desirable for the container to have a different shape, only the extensions (5) would need to be adapted and the movement of the seal (4) predicted, depending on the embodiment.

Distilled water or another innocuous liquid can be placed in the upper part, which helps the sample to detach from the clamps of the medical assistant. If it was desirable to maintain that part dry in order to avoid accidental spillages (which would prevent guaranteeing the final concentration of the product), a transversal protrusion (11) could be arranged in one or more extensions (5) in order to help the medical assistant to release the sample (Figure 2, left). The protrusion (11) will be thin enough to be able to enter between the arms of a clamp, which is preferably closed. For example, it can be 3 mm wide.

When a container has not yet been used, and in order to prevent contamination or the accidental release of the liquid from the upper part, the stopper (3) could have an extension or seal (12) (Figure 3, left) that allows the stopper (3) to be placed in the container (1) without affecting the extensions (5). Normally, the seal (12) will break when the stopper (3) is completely placed in the container. This seal (12) also ensures that the interior of the container is not contaminated and prevents any hypothetical vapours of the product from escaping.

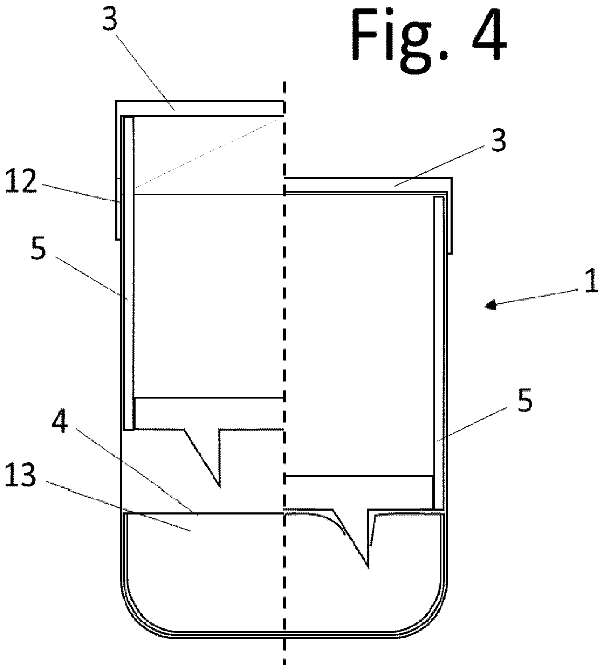

Figure 4 shows a new embodiment in which the product is placed inside a cartridge (13) or a removable watertight container in order to ensure that it is watertight. In this embodiment, the seal (4) is a sheet that breaks with one or more points (5') on the extensions (5). This solution makes it possible to guarantee the purity of the product inside the cartridge (13) from the time of manufacturing thereof.

The shape, number and dimensions of the extensions are not relevant so long as they achieve the desired outcome. For example, in a cylindrical container (1), the extensions (5) may correspond to a tube resting on the entire perimeter of the seal (4). The extensions (5), according to the type of embodiment, can be joined to the seal (4) or be independent. In every case, it should be understood that the extensions (5), despite using the plural and how they are represented in the drawings, may correspond to a single extension (5) with enough resistance and efficiency.

The shape of the container is also of little relevance. It has been represented as having cylindrical walls, since it is the simplest solution. The bottom of the container (1) could also have different shapes (conical, straight..., with or without a skirting). In the same way, the cartridge (13) can have different shapes and comprise elements fixing it to the bottom of the container (1) or not.

The materials used for all the elements makes no difference, so long as they resist the mechanical and chemical conditions they are being used in, and so long as they do not release any kind of additive that may affect the results.

## Claims

1. Sanitary container comprised of one container (1) with a mouth (2) and a stopper (3), **characterised in that** it comprises a seal (4) in an intermediate point of the container, defining a lower part designed to contain a product watertight, and also comprising at least one extension (5) protruding from the mouth (2), which is configured to render the seal (4) useless when the stopper (3) is placed.

2. Sanitary container, according to claim 1, where the extension (5) comprises a thin transversal protrusion (11) on the mouth of the container (1).

3. Sanitary container, according to claim 1, with a stopper (3) comprising an extension or seal (12) configured to become coupled to the container (1) without affecting the extensions.

4. Sanitary container, according to claim 1, with a seal (4) comprising a series of through-holes (9) blocked by a flexible sheet (8) adhered weakly from the upper part thereof.

5. Sanitary container, according to claim 1, with a seal (4) comprising at least one weak spot (6) and with the bottom of the container (1) comprising a projection (17) that prevents the seal (4) from moving downwards to a different area of the extension (5).

6. Sanitary container, according to claim 1, with a seal (4) that is immobile in the container (1) and that is configured so that the movement of the extensions (5) creates a space (10) for the seal to go through (4).

7. Sanitary container, according to claim 1, **characterised in that** the product is inside a removable cartridge (13) closed by the seal (4).
